# EUROPEAN PATENT APPLICATION

(11) **EP 1 403 385 A1**
(43) Date of publication of application: **31.03.2004**
(21) Application number: 02356188.9
(22) Date of filing: 27.09.2002
(51) Int. Cl.: C12Q 1/70

(54) **Method for assaying replication of HBV and testing susceptibility to drugs**

(71) Applicant: INSTITUT NATIONAL DE LA SANTE ET DE LA RECHERCHE MEDICALE (INSERM), 75654 Paris Cédex 13 (FR)
(72) Inventor: Durantel, David, 69003 Lyon (FR); Durantel, Sandra, 69003 Lyon (FR); Trepo, Christian, 69500 Bron (FR); Zoulim, Fabien, 69570 Dardilly (FR)
(74) Representative: Colombet, Alain André

(57) **Abstract**

A method for measuring the replication capacity of HBV, e.g. HBV present in a biological sample, possibly in the presence of a pharmaceutical product, in particular an antiviral agent, the method comprising:
(a) possibly extracting nucleic acids contained in the biological sample;
(b) PCR amplification of HBV nucleic acids using at least two primer pairs to obtain at least two amplified HBV genomic fragments representing more-than-full-length HBV genome;
(c) cloning the fragments obtained under (b) into a vector, so producing a vector containing more-than-full-length HBV DNA;
(d) transfecting or transducing susceptible cells with the vector;
(e) culturing the transfected or transduced cells in conditions allowing synthesis of HBV pgRNA from the cloned HBV DNA;
(f) possibly treating the cultured cells with the pharmaceutical product, in particular antiviral agent; and
(g) determination of the replication capacity of the HBV, possibly incidence of the pharmaceutical product, in particular antiviral agent, on viral gene expression and/or viral replication.

SEQUENCE LISTING

<110> INSTITUT NATIONAL DE LA SANTE ET DE LA RECHERCHE MEDICALE
(INSERM)

<120> Method for assaying replication of HBV and testing suscept
ibility to drugs

<130> BET 02/0923

<140> EP 02356188.9
<141> 2002-09-27

<160> 29

<170> PatentIn version 3.1

<210> 1
<211> 36
<212> DNA
<213> artificial sequence

<220>
<223> oligonucleotide

<400> 1
tgcgcaccgc ggccgcgcaa ctttttcacc tctgcc
36

<210> 2
<211> 36
<212> DNA
<213> artificial sequence

<220>
<223> oligonucleotide

<400> 2
tgcgcaccag ggcgcgccaa ctttttcacc tctgcc
36

<210> 3
<211> 36
<212> DNA
<213> artificial sequence

<220>
<223> oligonucleotide

<400> 3

tgcgcacccc tgcagggcaa ctttttcacc tctgcc
36

<210> 4
<211> 36
<212> DNA
<213> artificial sequence

<220>
<223> oligonucleotide

<400> 4
tgcgcaccag gtttaaacaa ctttttcacc tctgcc
36

<210> 5
<211> 36
<212> DNA
<213> artificial sequence

<220>
<223> oligonucleotide

<400> 5
tgcgcaccag cggccgccaa ctttttcacc tctgcc
36

<210> 6
<211> 36
<212> DNA
<213> artificial sequence

<220>
<223> oligonucleotide

<400> 6
tgcgcaccac ctgcaggcaa ctttttcacc tctgcc
36

<210> 7
<211> 36
<212> DNA
<213> artificial sequence

<220>
<223> oligonucleotide

<400> 7

tgcgcaccag gtttaaacaa ctttttcacc tctgcc
36

<210> 8
<211> 36
<212> DNA
<213> artificial sequence

<220>
<223> oligonucleotide

<400> 8
tgcgcaccag gcgcgcccaa ctttttcacc tctgcc
36

<210> 9
<211> 36
<212> DNA
<213> artificial sequence

<220>
<223> oligonucleotide

<400> 9
tgcgcacggc gcgcctgcaa ctttttcacc tctgcc
36

<210> 10
<211> 36
<212> DNA
<213> artificial sequence

<220>
<223> oligonucleotide

<400> 10
tgcgcaccct gcaggtgcaa ctttttcacc tctgcc
36

<210> 11
<211> 36
<212> DNA
<213> artificial sequence

<220>
<223> oligonucleotide

<400> 11

tgcgcaccgc ggccgcgcaa ctttttcacc tctgcc
36

<210> 12
<211> 36
<212> DNA
<213> artificial sequence

<220>
<223> oligonucleotide

<400> 12
tgcgcaccat taattaacaa ctttttcacc tctgcc
36

<210> 13
<211> 24
<212> DNA
<213> artificial sequence

<220>
<223> oligonucleotide

<400> 13
ggcagcacas cctagcagcc atgg
24

<210> 14
<211> 24
<212> DNA
<213> artificial sequence

<220>
<223> oligonucleotide

<400> 14
ggcagcacas ccgagcagcc atgg
24

<210> 15
<211> 23
<212> DNA
<213> artificial sequence

<220>
<223> oligonucleotide

<400> 15

acmtcstttc catggctgct agg
23

<210> 16
<211> 23
<212> DNA
<213> artificial sequence

<220>
<223> oligonucleotide

<400> 16
acmtcstttc catggctgct cgg
23

<210> 17
<211> 30
<212> DNA
<213> artificial sequence

<220>
<223> oligonucleotide

<400> 17
ctaagggcat gcgatacaga gcwgaggcgg
30

<210> 18
<211> 30
<212> DNA
<213> artificial sequence

<220>
<223> oligonucleotide

<400> 18
ctaagggtcg acgatacaga gcwgaggcgg
30

<210> 19
<211> 30
<212> DNA
<213> artificial sequence

<220>
<223> oligonucleotide

<400> 19

taaacaatgc atgaaccttt accccgttgc
30

<210> 20
<211> 43
<212> DNA
<213> artificial sequence

<220>
<223> oligonucleotide

<400> 20
ccggaaagct tatgctcttc tttttcacct ctgcctaatc atc
43

<210> 21
<211> 42
<212> DNA
<213> artificial sequence

<220>
<223> oligonucleotide

<400> 21
ccggagagct catgctcttc aaaaagttgc atggtgctgg tg
42

<210> 22
<211> 30
<212> DNA
<213> artificial sequence

<220>
<223> oligonucleotide

<400> 22
gctcttcttt ttcacctctg cctaatcatc
30

<210> 23
<211> 29
<212> DNA
<213> artificial sequence

<220>
<223> oligonucleotide

<400> 23

gctcttcaaa aagttgcatg gtgctggtg
     29

<210>  24
<211>  42
<212>  DNA
<213>  chicken

<400>  24
cggccctata aaaagcgaag cgcgcggcgg gcgggagtcg ct
     42

<210>  25
<211>  34
<212>  DNA
<213>  cytomegalovirus

<400>  25
tatataagca gagctcgttt agtgaaccgt caga
     34

<210>  26
<211>  34
<212>  DNA
<213>  human

<400>  26
tatataagga cgcgccgggt gtggcacagc tagt
     34

<210>  27
<211>  33
<212>  DNA
<213>  human

<400>  27
tatataagtg cagtagtcgc cgtgaacgtt ctt
     33

<210>  28
<211>  34
<212>  DNA
<213>  SV40 virus

<400>  28
gactaatttt ttttatttat gcagaggccg aggc
     34

<210> 29
<211> 33
<212> DNA
<213> Rous Sarcoma virus

<400> 29
tatttaagtg cctagctcga tacaataaac gcc
33

## Description

The present invention relates to the cloning of replication-competent HBV DNA genomes, to methods for measuring the replication capacity of HBV, e.g. HBV present in a biological sample, possibly in the presence of a pharmaceutical product, in particular an antiviral agent. The invention relates in particular to phenotypic assays allowing to test the susceptibility of HBV to drugs or to screen drugs on HBV.

More particularly, the invention concerns such methods to determine the susceptibility of the viral strains present in a patient to the available drugs and therefore tailor antiviral therapy to the virological situation.

The invention also relates to primers, primers pairs and kits used in amplification, as well as to vectors comprising replication-competent HBV DNA genomes and cells, e.g. cell lines transfected with such vectors.

Despite the development of an efficient vaccine, chronic Hepatitis B virus (HBV) infection is still a major public health problem worldwide. Indeed, more than 400 million people are chronic carriers of the virus and are at high risk of developing cirrhosis and hepatocellular carcinoma.

HBV is a DNA virus that replicates its genome via a reverse transcriptase phase. The spontaneous error rate of the viral reverse transcriptase leads to the heterogeneity of HBV genome. Illustrating the latter point, seven genotypes (A to G) have been identified, and HBV mutants which are selected either during the natural course of the infection or during therapy, have been described.

Recently, major progress has been made in the field of antiviral therapy of chronic hepatitis B, with the development of nucleoside analogues that inhibit the viral polymerase. Hence, lamivudine has been approved two years ago and is now largely used in clinical practice worldwide. Although it inhibits significantly viral replication, the emergence of drug resistant mutants harbouring mutations in conserved domains of the viral polymerase is observed with a prevalence in patient increasing from 15 at year one to 75 % at year four of therapy. As these mutants may represent a new threat for human health, new antiviral agents were needed to combat them. Thus, other inhibitors, such as adefovir dipivoxil, entecavir, emtricitabine, clevudine, and telbivudine, have been recently studied for their anti-HBV properties in experimental models and are currently evaluated in clinical trials. However, *in vitro* studies have also shown that lamivudine resistant strains may be cross-resistant to some of these drugs (e.g. emtricitabine), while sensitive to other. This observation warrants a close monitoring of viral population *in vivo* and emphasises the development of drug resistance assay (or phenotypic assay).

Therefore, it will be important in the future, in the clinical setting, to know in each patient the susceptibility of the viral strains to the available drugs and therefore tailor antiviral therapy to the virological situation.

To date, no phenotypic drug susceptibility assay has been developed for HBV. With the current and future development of new anti-HBV molecules, phenotypic drug susceptibility testing should become an important issue for the management of patient infected with resistant HBV isolates.

The mechanism of HBV entry into cells has not been completely elucidated. After uncoating, the HBV DNA is brought into the nucleus of the host cell, where it is repaired to the covalently closed circular form (cccDNA). Once recircularized, enhancer and promoter activities start producing the various HBV RNA transcripts. There are sub-genomic transcripts which serve as mRNA for the expression of the X and the surface proteins. The larger genomic transcripts are longer than one genome in length and serve to produce e, core and polymerase proteins. And a particular genomic transcript, lacking the ATG start codon for the e protein, is designated the pregenomic RNA (pgRNA). The transcription of this pgRNA results in the generation of a terminally-redundant RNA strand, which will be then converted into DNA by the polymerase. The redundant sequence comprises about 200 nucleotides, including direct repeat 1 (DR1) as well as the stem-loop. See for more details G-H Wang et al., Cell 1992, 71: 663-670, G-H Wang et al., J. Virol. 1993, 67: 6507-6512).

Günther *et al(*J*.* Virol. 1995, 69, 9: 5437-5444) have described a method for PCR amplification of full-length HBV genomes that was meant to facilitate the analysis of natural HBV isolates. The full-length amplicon was either directly used for transfection experiment of hepatoma cell line or cloned prior to its use. Both approaches involved the utilization of Sap-I, which is a quite expensive and inefficient restriction enzyme. Other limitations are the high amounts of amplification products that are necessary for cell transfection, and the absence of "an easy to use" continuous source of material for cell transfections and phenotypic studies. Although interesting for fundamental research, this method does not seem appropriate in the context of a standardized phenotypic assay which requires solid replication level and production of easily detectable amount of viral particles in the cell culture medium.

Other more classic methods rely on site directed mutagenesis of replication competent HBV constructs or on the introduction of HBV genome fragments from clinical isolates in replication competent HBV constructs. However these methods do not allow to studying the whole HBV genetic information from the clinical isolates and may therefore exclude some important mutations located in other parts of the genome or result in a mosaic HBV genome.

There is thus still a need for an efficient method allowing to evaluate the replication capacity of HBV and to test the susceptibility of HBV isolates to drugs and/or to test the antiviral activity of drugs on HBV.

An objective of the invention is to propose a rapid and reproducible cloning of replication-competent HBV DNA genomes from patient samples.

Another objective of the invention is to propose a rapid and reproducible phenotypic assay method allowing to test the susceptibility of HBV isolates with respect to drugs. In particular, this object aims to determine the susceptibility of the viral strains present in a patient to the available drugs and therefore tailor antiviral therapy to the virological situation.

Another objective of the invention is to propose a method allowing to rapidly produce *in vitro* HBV replication competent clones useful for phenotypic analysis or for functional analysis.

Still another objective of the invention is to propose a rapid and reproducible assay method allowing to test the antiviral activity of current and future drugs to particular HBV or to HBV variants or to HBV field isolates.

Still another objective is to propose a method allowing to study the replication capacity of wild-types and mutants of HBV

Still another objective is to allow the production of cell lines constitutively producing HBV particles.

The present invention is based on the cloning into a vector of HBV DNA covering more-than-full-length HBV genome, so that a pgRNA can be synthesized from this DNA post-cell-transfection. The pgRNA synthesis in the host cells initiates the HBV intracellular cycle with viral gene expression, including synthesis of intracellular replicative intermediates, which comprise single stranded DNA, double stranded linear DNA, relaxed circular DNA and the minor species of replicative intermediates. The invention allows the rapid cloning of replication-competent HBV DNA genomes. Then, it is possible to evaluate the level of HBV DNA synthesis in the cells, e.g. by Southern blot, or of RNAs synthesis, e.g. by Northern blot, or of viral protein expression, e.g. by Western blot, ELISA or RIA, in the presence or in the absence of antiviral agent to be evaluated. Is also possible to analyse the level of virus production, in particular by measuring the level of virion DNA or of Dane particles in the extra-cellular medium or supernatant, using Southern blot or real time PCR method or similar, in the presence or in the absence of antiviral agent to be evaluated.

A first object of the invention is thus a method for measuring the replication capacity of HBV, e.g. HBV present in a biological sample, possibly in the presence of a pharmaceutical product, in particular an antiviral agent, the method comprising:
(a) possibly extracting nucleic acids contained in the biological sample;
(b) PCR amplification of HBV nucleic acids using at least two primer pairs to obtain at least two amplified HBV genomic fragments representing more-than-full-length HBV genome;
(c) cloning the fragments obtained under (b) into a vector, so producing a vector containing more-than-full-length HBV DNA;
(d) transfecting or transducing susceptible cells with the vector;
(e) culturing the transfected or transduced cells in conditions allowing synthesis of HBV pgRNA from the cloned HBV DNA;
(f) possibly treating the cultured cells with the pharmaceutical product, in particular antiviral agent; and
(g) determination of the replication capacity of the HBV, possibly incidence of the pharmaceutical product, in particular antiviral agent, on viral gene expression and/or viral replication.

The biological sample may be derived from a patient. It can be a sample of blood or serum, but can be any body fluid or else a liver biopsy sample, or any preparation issued therefrom. The biological sample may also be derived from a viral culture. It can be a cell culture supernatant. Generally speaking a biological sample can be any type of sample containing an HBV.

Step (a) provides the extraction of DNA from the biological sample. This can be made by any usual method. These methods may comprise a chromatography column-based purification, in particular affinity chromatography, and/or differential precipitation of proteins and nucleic acids. Use may be made of commercial purification kits, as those used in the examples.

The cloning of a HBV DNA covering more-than-full-length HBV genome is done by amplifying (step (b)) at least two, preferably two, HBV genomic fragments, then subjecting the amplicons with suitable restriction enzymes to recombine the whole insert in the vector (step (c)).

Each HBV genomic fragment is amplified in step (b) using suitable primer pairs.

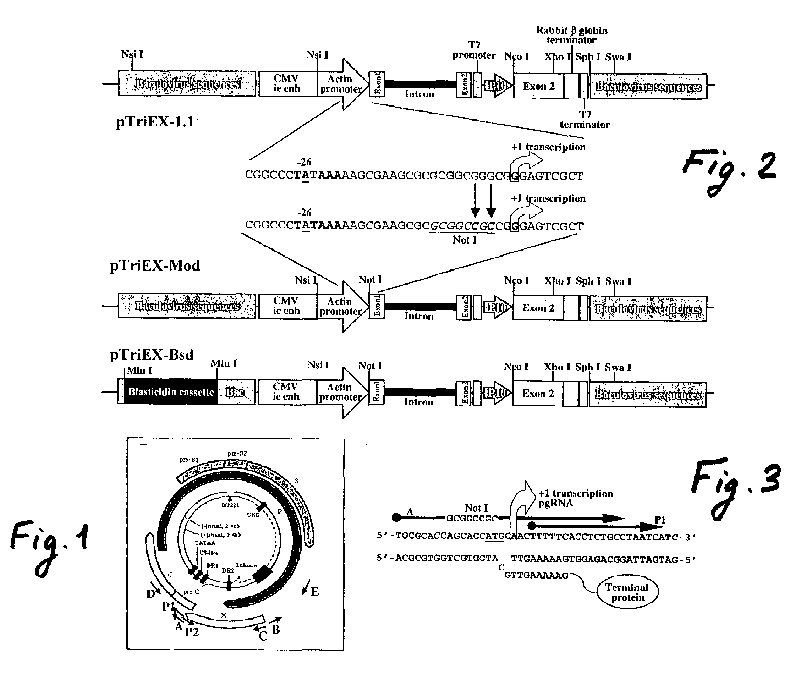

The HBV are classified into the genetic groups A to G based on divergence of the complete nucleotide sequence. Such sequences are available in articles and in GenBank for each genotype. In the present specification, the numbering of nucleotides is by reference to the whole HBV genomic DNA sequence published in H. Norder et al. (Virology 1994, 198, 489-503; Figure 1 of this article presents an alignment of the genome of various HBV representing genotypes C, E and F, with the sequence of clone pHBV-3200 which is 3221 nucleotides long) or the one of a genotype C HBV published in GenBank under access number AB048704. The length of the genome of the various HBV is variable. That is to say that numbering of nucleotides should only be considered as illustrative embodiments.

In a first embodiment, the aim is to clone a more-than-full-length HBV genome under the control of an heterologous promoter. In this embodiment, by definition, a more-than-full-length HBV genome comprises HBV nucleotides (from 5' to 3') :
- an about 1 unit genome starting in 5' from and including the nucleotide representing the +1 of transcription (in general nucleotide A, position 1818 in H. Norder at al.; in some cases however, the +1 of transcription has revealed to be the nucleotide in 5' of said A, i.e. C, or in 3', i.e. A) to the first nucleotide in 5' of the ATG of the pre-C gene (nucleotide 1813), plus
- a redundant sub-genomic fragment starting from and including the A of the ATG of the pre-C gene (nucleotide 1814) and extending to and including the polyA attachment site (nucleotide 1960), thus comprising the polyA sequence TATAAA, nucleotides 1916-1920).

If one refers to H. Norder at al. or in GenBank AB048704, the more-than-full-length HBV genome comprises from 5' to 3' nucleotides 1818 to 1813 and 1814 to 1960. The number of nucleotides may be variable depending on the HBV. For instance, some HBV lack several nucleotides at positions 2806 to 2611 and 2884 to 2886 (Norder et al., Figure 1). It is however evident that this teaching extends itself to any HBV. In one embodiment, the more-than-full-length HBV genome comprises from 5' to 3' nucleotides 1818 to 1813 and 1814 to 1960 of an HBV genomic sequence aligned with the one disclosed in H. Norder at al. or in GenBank AB048704 (alignment means that spaces are introduced for the missing nucleotides, as was done for instance in Norder et al., or that possible supplemental nucleotides are not considered). In a particular embodiment, it comprises nucleotides 1816 to 1813 and 1814 to 2016 of an HBV genomic sequence aligned with the sequence as mentioned above.

Also, the more-than-full-length HBV genome can be longer and for instance can comprise further nucleotides situated in 5' of the said +1 of transcription. In that event however, it is necessary that the longer sequence does not comprise in 5' a functional ATG for the pre-C gene (A and/or T and/or G mutated or deleted). Also, it can be longer at the 3' end. The location of the primer determines the length of the sequence. It is preferred to choose a primer which is complementary to a well-conserved region inside HBV genotypes and as close as possible of the polyA attachment site. The sensibility of the PCR is better with smaller amplicons.

In this first embodiment, amplification is made of two fragments whose DNA sequence comprises HBV nucleotides (from 5' to 3') :
- the first fragment comprises a HBV DNA sequence comprising at its 5' end the nucleotide representing the +1 of transcription, and
- the second fragment comprises a HBV DNA sequence comprising at its 3' end the polyA attachment site
the 3' end of the first fragment and the 5' end of the second fragment being overlapping and preferably comprising a restriction site in the overlapping part, so that upon restriction treatment both fragments can be ligated to give access to the more-than-full-length HBV genome.

Two primers pairs are designed to amplify such first and second primers, respectively.

The primers disclosed herein have been designed after multiple sequence alignment with more than 200 whole HBV genomes. As it is the case with the herein disclosed primers, primers useful in the invention are better designed from well-conserved regions, in order to allows one to apply the invention regardless of the genotypes and main natural mutations of the field HBV. In order to allow representing most if not all HBV genomes, an embodiment provides for the use of a mixture of different primers which differ only by the variations necessary to represent all possibilities. For instance, if the HBV genomes can be classified into two families regarding a specific genomic region targeted by the primer, the primer designed to be complementary to this region may be a mixture of two primers, representing each one of these groups.

In this first embodiment, a preferable first primer pair comprises a forward primer A which is partially complementary to the 5' part of the pre-C gene (which codes for protein e), including the nucleotide representing the +1 of transcription (as defined above), but does not contain the ATG of the pre-C gene.

Suitable A primers are by way of example: 
SEQ ID NO: 1
5' TGCGCACCGCGGCCGCGCAACTTTTTCACCTCTGCC 3'
 This primer comprises a Notl site (once underlined) downstream of the G of the start codon of pre-C, and hybridise on nucleotides 1816 to 1835 (H. Norder et al., and GenBank AB048704). 
SEQ ID NO: 2
5' TGCGCACCAGGGCGCGCCAACTTTTTCACCTCTGCC 3'
 This primer comprises a *Asc*l site (once underlined). 
SEQ ID NO: 3
5' TGCGCACCCCTGCAGGGCAACTTTTTCACCTCTGCC 3'
 This primer comprises a Sse8387 l site (once underlined). 
SEQ ID NO: 4
5' TGCGCACCAGGTTTAAACAACTTTTTCACCTCTGCC 3'
 This primer comprises a Pmel site (once underlined). 
SEQ ID NO: 5
5' TGCGCACCAGCGGCCGCCAACTTTTTCACCTCTGCC 3'
 This primer comprises a *Not*l site (once underlined). 
SEQ ID NO: 6
5' TGCGCACCACCTGCAGGCAACTTTTTCACCTCTGCC 3'
 This primer comprises a *Sse8387* l site (once underlined). 
SEQ ID NO: 7
5' TGCGCACCAGGTTTAAACAACTTTTTCACCTCTGCC 3'
 This primer comprises a Pmel site (once underlined). 
SEQ ID NO: 8
5' TGCGCACCAGGCGCGCCCAACTTTTTCACCTCTGCC 3'
 This primer comprises a *Asc*l site (once underlined). 
SEQ ID NO: 9
5' TGCGCACGGCGCGCCTGCAACTTTTTCACCTCTGCC 3'
 This primer comprises a *Asc*l site (once underlined). 
SEQ ID NO: 10
5' TGCGCACCCTGCAGGTGCAACTTTTTCACCTCTGCC 3'
 This primer comprises a *Sse8387*l site (once underlined). 
SEQ ID NO: 11
5' TGCGCACCGCGGCCGCGCAACTTTTTCACCTCTGCC 3'
 This primer comprises a *Not*l site (once underlined). 
SEQ ID NO: 12
5' TGCGCACCATTAATTAACAACTTTTTCACCTCTGCC 3'
 This primer comprises a *Pac*l site (once underlined).

In this first embodiment, it is preferred to clone HBV DNA in order to fuse the natural +1 of transcription of the HBV pgRNA to the +1 of transcription determined by the promoter contained in the vector or plasmid used for the cloning. To achieve this fusion at molecular level, the promoter may be first modified by the insertion (mutagenesis), between the TATA box (or equivalent) of the basic promoter and the +1 of transcription, of a restriction site which is preferably not present in any of the known complete HBV sequences. Several restriction enzymes that recognize a palindromic site of 8 nucleotides, such as *Not*l*, Asc*l*, Pac*l*, Pme*l*, SSe8387*l, do not cut HBV genome, and can be used for this strategy. The same restriction site is also integrated into the tail of the primer A used for the PCR. SEQ ID NO: 1 to 12 are examples of primers useful in this embodiment, comprising such restriction sites. Of course, it is evident that the one skilled in the art is able to prepare other suitable primers.

Generally speaking, polynucleotides useful as preferable primers in this embodiment comprise a nucleotide sequence corresponding to a restriction site, preferably not present in the HBV genome, situated in 5' of a sequence complementary to the 5' end of the pre-C gene, but lacking the complete ATG of this pre-C gene (e.g. A, or A and T or A and T and G are missing). As appearing on the sequences referred SEQ ID NO: 1 to 12, the restriction site can be directly linked to the +1 of transcription or separated by some nucleotides. In the preferred embodiment, the primer comprises SEQ ID NO: 1. On the sequences, the double underlined sequence is complementary to the 5' part of the pre-C gene. Of course, the 3' end thereof may be shortened or extended to contain further complementary nucleotides. If one wants to keep the possibility of hybridising DNA from any HBV, it is preferable to not extend the primer so that it includes non-conservative regions.

This first primer pair comprises preferably a reverse primer chosen to be complementary to a consensus or a substantially consensus sequence within the HBV genomes.

Suitable reverse B primers are by way of example: 
B1: SEQ ID NO: 13
5' GGCAGCACASCCTAGCAGCCATGG 3'
 This primer comprises a Ncol site (underlined) and are useful for amplification of genotypes A, B, D, E and G. The primer hybridizes with nucleotides 1395-1372 (H. Norder et al., and GenBank AB048704). 
B2: SEQ ID NO: 14
5' GGCAGCACASCCGAGCAGCCATGG 3'
 This primer comprises a *Nco*l site (underlined) and are useful for amplification of genotypes C and F. The primer hybridizes with nucleotides 1395-1372.

A mixture of both primers B1 and B2 may advantageously be used.

S means G or C. Thus in SEQ ID NO: 13 and 14, S can represent G or C. A mixture of both can be used. Generally, the primers will be synthesized with degeneracy, and will thus comprise both possibilities.

The second primer pair comprises preferably a forward primer chosen to be complementary to a consensus or a substantially consensus sequence within the HBV genomes; this primer is designed to be complementary to a region of HBV DNA which is in 5' with respect to that complementary with the reverse primer of the first primer pair. An example of such a forward primer is primer E described herein after and having the sequence as set forth in SEQ ID NO: 19. The best is to have both these forward and reverse primers complementary to HBV genomic regions which overlap and comprise a unique natural restriction site, e.g. the *Nco*l site at nucleotides 1372-1377.

Suitable forward primers are by way of example: 
C1: SEQ ID NO: 15
5' ACMTCSTTTCCATGGCTGCTAGG 3'
 This primer comprises a *Nco*l site (underlined) and are useful for amplification of genotypes A, B, D, E and G. The primer hybridizes with nucleotides 1363-1385 (H. Norder et al., and GenBank AB048704). 
C2: SEQ ID NO: 16
5' ACMTCSTTTCCATGGCTGCTCGG 3'
 This primer comprises a *Nco*l site (underlined) and are useful for amplification of genotypes C and F. The primer hybridizes with nucleotides 1363-1385.

A mixture of both primers C1 and C2 may advantageously be used.

M means A or C, and W means A or T. Thus in SEQ ID NO: 15 and 16, M can represent A or C, and W can represent A or T. A mixture can be used. Generally, the primers will be synthesized with degeneracy, and will thus comprise the various possibilities.

The second primer pair comprises preferably a suitable reverse primer chosen to be complementary to a consensus or a substantially consensus sequence within the HBV genomes; this primer is designed to be complementary to a region of the HBV genome which is in 3' with respect to that of the forward primer in the first pair of primers and more precisely in 3' of the polyA attachment site. As a result, the cloned DNA obtained after recombination between both amplified fragments or amplicons, is longer than the HBV genome and include a redundant region including the start codon ATG of the pre-C gene and the polyA attachment site. As mentioned above, it is preferred to choose a primer which is complementary to a well-conserved region inside HBV genotypes and as close as possible in 3' of the polyA attachment site.

Suitable reverse primers D are by way of example: 
D1: SEQ ID NO: 17
5' CTAAGGGCATGCGATACAGAGCWGAGGCGG 3'
 This primer comprises a *Sph*l site (underlined). The 3' part of the primer hybridizes with nucleotides 2027-1998. The C at the 5' end corresponds to nucleotide 2039. 
D2: SEQ ID NO: 18
5' CTAAGGGTCGACGATACAGAGCWGAGGCGG 3'
 This primer comprises a *Sal*l site (underlined). The 3' part of the primer hybridizes with nucleotides 2027-1998. The C at the 5' end corresponds to nucleotide 2039.

A mixture of both primers D1 and D2 may advantageously be used.

W means A or T. Thus in SEQ ID NO: 17 and 18, W can represent A or T. A mixture can be used. Generally, the primers will be synthesized with degeneracy, and both possibilities will be present.

In a second embodiment, the aim is to clone a more-than-full-length HBV genome under the control of homologous enhancer and promoter, thus comprising the HBV nucleotide sequence of enhancer 1 (EN 1 or enh 1, nucleotides 1180 to 1219, H. Norder et al., and GenBank AB048704), and ending on or after a polyA attachment site. Before cloning, preferably two HBV genome fragments have to be amplified.

A first primer pair comprises a forward primer E which preferably is complementary to a consensus or a substantially consensus sequence within the HBV genomes, this primer being designed to be complementary to a region of the HBV genome which is in 5' of enhancer 1, preferably as close as possible of this enhancer.

By way of example, this forward primer E can be as set forth in 
SEQ ID NO: 19
5' TAAACAATGCATGAACCTTTACCCCGTTGC 3'
 This primer comprises a *Nsi*l site (underlined). The primer hybridizes with nucleotides 1125-1154 (H. Norder et al., and GenBank AB048704).

The first primer pair comprises a reverse primer chosen to be complementary to a consensus or a substantially consensus sequence within the HBV genomes. The above described reverse primer D can be used, e.g. either D1 or D2 or D1 and D2, complementary to a region situated in 3' of the polyA attachment site.

Primers E and D allow to amplify a genomic fragment containing the enhancer 1, a unique Ncol site which is situated at the level of the ATG of the X gene, and the polyA attachment site (position 1372-1377 in Norder et al. and GenBank AB048704).

The second primer pair comprises a forward primer and a reverse primer allowing to amplify an about one genome unit containing the same *Nco*l site, with a forward primer designed to be complementary to a region of HBV DNA which is in 5' with respect to that complementary with the reverse primer of the first primer pair, and a reverse primer designed to be complementary to a region of HBV DNA which is in 3' with respect to that complementary with the forward primer of the first primer pair.

By way of example, use can be made of primers comprising the sequences SEQ ID NO: 20 (forward primer) and SEQ ID NO: 21 (reverse primer) described in the examples, or the following primers:

### Forward primer

SEQ ID NO: 22
5' GCTCTTCTTTTTCACCTCTGCCTAATCATC 3'

containing a *Sap*l site; hybridizes with nucleotides 1821 to 1843.

### Reverse primer

SEQ ID NO: 23
5' GCTCTTCAAAAAGTTGCATGGTGCTGGTG 3'

containing a *Sap*l site; hybridizes with nucleotides 1825 to 1804.
Preferably, these primers SEQ ID NO: 22 and 23 comprise further nucleotides at the 5' end, in particular 8 or more, e.g. 8 to 15. This is to facilitate the action of the restriction enzyme.

All nucleotide sequences or polynucleotides and primers, as well as primers pairs, described above are also objects of the invention.

In an embodiment, the invention concerns a primers pair for HBV amplification comprising a forward primer comprising (1) a sequence as set forth in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 or SEQ ID NO: 12, and (2) a reverse primer comprising a sequence as set forth in SEQ ID NO: 13 or SEQ ID NO: 14 or a mixture of a reverse primer comprising a sequence as set forth in SEQ ID NO: 13 and a reverse primer comprising a sequence as set forth in SEQ ID NO: 14.

In another embodiment, the invention concerns a primers pair for HBV amplification comprising (1) a forward primer comprising a sequence as set forth in SEQ ID NO: 15 or SEQ ID NO: 16 or SEQ ID NO: 19, or a mixture of a forward primer comprising a sequence as set forth in SEQ ID NO: 15 and a forward primer comprising a sequence as set forth in SEQ ID NO: 16, and (2) a reverse primer comprising a sequence as set forth in SEQ ID NO: 17 or SEQ ID NO: 18 or a mixture of a reverse primer comprising a sequence as set forth in SEQ ID NO: 17 and a reverse primer comprising a sequence as set forth in SEQ ID NO: 18.

The invention also relates to kits for amplifying HBV, comprising two primer pairs according to the invention. In an embodiment, the kit comprises both primers pairs described just before.

In still other embodiment, the invention concerns a primers pair for HBV amplification comprising (1) a forward primer comprising a sequence as set forth in SEQ ID NO: 19, and (2) a reverse primer comprising a sequence as set forth in SEQ ID NO: 17 or SEQ ID NO: 18 or a mixture of a reverse primer comprising a sequence as set forth in SEQ ID NO: 17 and a reverse primer comprising a sequence as set forth in SEQ ID NO: 18.

In still other embodiments, the invention concerns a primers pair for HBV amplification comprising (1) a forward primer comprising a sequence as set forth in SEQ ID NO: 20 or 22, and (2) a reverse primer comprising a sequence as set forth in SEQ ID NO: 21 or 23.

The invention also relates to a kit comprising both primers pairs described just before.

The one skilled in the art is able to design other primers. Following the same scheme he is also able to amplify more than two fragments, e.g. 3 fragments, using the corresponding number of primer pairs, and to clone the more-than-full-length DNA from these fragments.

And the one skilled in the art is also able to determine the best conditions for the amplifications, depending on the primers which are used. The hybridisation temperature may be determined owing the usual method based on the determination of the dissociation temperature. Also, generally speaking, the amplification may involve a number of cycles, e.g. from 20 to 60, in particular from 30 to 50, preferably about 40 cycles. The Taq polymerase, preferably High Fidelity Taq polymerase (e.g. Expand from Roche Diagnostics) may be used .

In step (c), the product of the amplification is cloned into a vector. This lead to a vector comprising HBV DNA covering more-than-full-length HBV genome, and elements controlling the synthesis of a pgRNA from the DNA sequence post-cell-transfection. Such a vector is also an object of the invention.

The vector comprises the elements necessary to govern synthesis of pgRNA from the DNA sequence. This comprises a promoter able to ensure the transcription of DNA into RNA in eucaryotic cells.

In the first embodiment, the promoter is an heterologous promoter operatively linked to the nucleotide representing the +1 of transcription of the pgRNA, as defined above. The vector called herein type-I vector does not comprise the ATG of the pre-C gene.

Potentially, vectors containing an ubiquitous mammalian promoter may be used for constructing a type-I vector.

In a preferred embodiment, the heterologous promoter is the early promoter of the cytomegalovirus or CMV-IE (Cytomegalovirus Immediate Early), in particular of human or murine origin, or optionally of any other origin such as rat or guinea pig. The CMV-IE promoter may comprise the proper promoter component, which may or may not be associated with the enhancer component. Use is preferably made of the complete promoter, including the enhancer. Reference may be made to EP-A-260 148, EP-A-323 597, US-A-5 168 062, US-A-5 385 839, US-A-4 968 615 and WO-A-87/03905, Boshart M. *et al.,* Cell., 1985, **41**, 521-530.

Other promoters include the human ubiquitine C gene promoter, the promoter of EF-1α gene, the early and late promoters of the SV40 virus (Simian Virus 40), the LTR promoter of the Rous sarcoma virus, cytoskeleton gene promoters, such as, the desmin promoter (Kwissa M. et *al.,* Vaccine, 2000, **18(22),** 2337-2344) or the actin promoter (Miyazaki J. et *al.,* Gene, 1989, **79(2),** 269-277), e.g. of the chicken β-actin gene, which is also a preferred choice (Fregien N, Davidson N. Gene 1986 48, 1-11 ).

The CMV-IE enhancer may be combined to a promoter of another origin, e.g. with the actin promoter, in particular the chicken β-actin gene

By equivalence, the sub-fragments of these promoters, maintaining adequate promoter activity, are included in the present invention: e.g. the truncated CMV-IE promoters according to WO-A-98/00166. The notion of promoter according to the invention therefore includes the derivatives and sub-fragments maintaining adequate promoter activity, preferably substantially similar to that of the proper promoter from which they are derived. With regard to CMV-IE, this notion includes the proper promoter part and/or the enhancer part and the derivatives and sub-fragments.

As explained above, it is preferred to fuse the natural +1 of transcription of the HBV pgRNA to the +1 of transcription determined by the promoter contained in the vector or plasmid used for the cloning. To achieve this fusion at molecular level, the promoter is modified by the insertion (mutagenesis), between the TATA box (or equivalent) of the basic promoter and its +1 of transcription, of a restriction site which preferably is not present in any of the known complete HBV sequences (e.g. one of the previously mentioned *Not*l*, Asc*l, *Pac*l*, Pmel* and *SSe8387*l restriction sites). The restriction site has to be the same than the one present in the primer A. See the examples.

The cloning necessitates the ligation of the HBV DNA fragments together, and insertion into the vector. This technique is well known to one skilled in the art using suitable sets of restriction enzymes and restriction sites. Preferably step (c) is a one-step cloning. And the primers specifically disclosed herein allow a rapid one-step cloning.

Using a type A primer, the vector comprises:
(a) a more-than-full length HBV genome according to the first embodiment
(b) a promoter modified in 5' by the presence of a restriction site in 5' of the +1 of transcription, with the +1 of transcription of the more-than-full length HBV genome and of the promoter being fused, the promoter controlling the synthesis of a pgRNA from the more-than-full length HBV genome post-cell-transfection. This restriction site is preferably not present in any of the known complete HBV sequences and can be e.g. *Not*l*, Asc*l*, Pac*l*, Pme*l or *SSe8387* I. In some particular embodiments, the promoter sequence comprises a sequence chosen among SEQ ID NO: 24 to 29 (end part of the specification), after putting the restriction site as indicated.

The vector may also comprise baculovirus sequences, allowing one to transfer the vector in baculovirus. These sequences are sequences flanking a baculovirus genome region, e.g. gene, which is not essential for viral replication in cell culture. These sequences allow recombination between the vector and the baculovirus genome, so that the cassette containing the promoter and the HBV unit becomes inserted in the baculovirus genome upon transfer of the vector into the baculovirus.

The vector can also comprise a selection marker, such as a resistance gene, e.g. a blasticidin resistance gene. This could be helpful in the course of generating stable cell lines, to select the cells containing the vector.

A vector comprising the baculovirus sequence and the selection marker is contemplated herein. The same vector could thus be used either to transfect cells, or to transduce cells via baculovirus, or to produce a stable line cell. In particular, once an interesting clone has been found from a patient sample, it is possible to produce a stable cell line in view of screening of efficient drugs.

The synthesis of a pgRNA initiates in the cells the HBV intracellular cycle with viral gene expression, including synthesis of intracellular replicative intermediates (which comprise single stranded DNA, double stranded linear DNA, relaxed circular DNA and the minor species of replicative intermediates, RNAs), gene expression and virus production.

Such vectors are also objects of the invention. Also, the promoters modified as described herein constitute other objects of the invention.

In the second embodiment, the promoter is an homologous promoter, which is present in the cloned HBV DNA. A type-II vector is produced in a two step procedure. First, the amplicon obtained with the first set of primers, e.g. E-D, is digested and inserted into the vector. Then, the amplicon obtained with the second set of primers, e.g. P1-P2, is digested by *Sap*l*,* ligated (circularized) using a ligase, then digested with *Nco*l and inserted in the *Nco*l site present in the cloned E-D fragment.

This strategy produce a vector comprising :
- a more-than-full length HBV genome according to the second embodiment
- wherein the homologous enhancer and promoter controls the synthesis of a pgRNA from the more-than-full length HBV genome post-cell-transfection. More precisely, the vector initiates synthesis of pre-C RNA and of pgRNA. Such vectors are also objects of the invention.

In step (d), cultured susceptible cells can be transfected or transduced with the vector according to the invention.

In one embodiment the susceptible cells, in particular eucaryotic cells such as hepatocytes, including cell lines, in particular cell lines from hepatocyte origin, e.g. hepatoma cell lines, e.g. Huh7 (Nakabayaski, H. et al.. Cancer Res.1982 42,3858-3863), HepG2 (available from the ATCC, access number HB-8065) are transfected with the vector.

At step (e), the cells are cultured to allow the vector to synthesize pgRNA and possibly initiate the HBV intracellular cycle as described previously.

In step (g), replication capacity of HBV is evaluated. This may involve measuring the level of nucleic acid synthesis, protein synthesis and/or virus production.

In one embodiment, the transfected cells may be used for studying the replication capacity of the HBV on the cells. This may involve quantification of viral DNA by molecular biology techniques, e.g. by Southern blot or by real time amplification specifically of the synthesized nucleic acids.

In another embodiment comprising step (f), these transfected cells are used for studying the effect of antiviral agents. In step (f), when a pharmaceutical product, in particular the antiviral agent, is used, it is added to the cultured transfected cells. In an embodiment, a part of the cultured transfected cells is kept as a control, say without being subjected to the pharmaceutical product, in particular the antiviral agent, in order to have a comparison basis. In another embodiment, one or more parts of the cultured transfected cells is/are subjected to another pharmaceutical product(s), in particular antiviral agent(s), which allows comparison between different pharmaceutical product(s), in particular antiviral agent(s). And in this embodiment, a control without any treatment may be added.

In particular embodiments, the antiviral agents used are chosen among lamivudine, adefovir dipivoxil, entecavir, emtricitabine, clevudine, telbivudine, tenofovir and any combinations thereof.

In still another embodiment, the vector is transferred inside a baculovirus upon infection of an appropriate systems of cells such as insect cells. One may use for example the Baculovirus Autographa californica Nuclear Polyhedrosis Virus AcNPV. One may use for example Spodoptera frugiperda Sf9 (ATCC CRL 1711 deposit) as insect cells. The baculovirus containing the vector is then used for transducing eucaryotic cells such as hepatoma cells, e.g. Huh7 cells, where the vector can initiate pgRNA synthesis. Then in step (f), a pharmaceutical product, in particular an antiviral agent, is added to the cultured transduced cells. In an embodiment, a portion of the cultured transduced cells is kept as a control, say without being subjected to the pharmaceutical product, in particular the antiviral agent, in order to have a comparison basis. In another embodiment, one or more portions of the cultured transduced cells is/are subjected to another pharmaceutical product(s), in particular antiviral agent(s), which allows comparison between different pharmaceutical product(s), in particular antiviral agent(s). And in this embodiment, a control without any treatment may be added.

In still another embodiment, the vector is used for generating cell lines which become able to constitutively produce HBV virions. As mentioned above, the vector may comprise a selection marker. Cell lines such as cell lines from hepatocyte origin, e.g. hepatoma cell lines, e.g. Huh7, HepG2 are transfected with the vector as mentioned above and the cells selected owing the marker are recovered as stable cell line comprising the vector according to the invention. These stable cell lines are also an object of the invention.

As explained before, the present invention results in producing HBV pgRNA, intracellular replicative intermediates, and HBV viral particles. It is thus possible in step (g) to follow different parameters, in the presence or in the absence of pharmaceutical product, in particular an antiviral agent.

In a first embodiment, the level of HBV DNA synthesis in the cells is evaluated and possibly compared. A general method provides the disruption of the cells, e.g. by physical and/or chemical treatment, for instance lysis, e.g. with Tris-EDTA and Nonidet P-40. The recovered sample is centrifuged, the recovered supernatant is subjected to DNase to eliminate transfected DNA, whereas the synthesized DNA or being synthesized is encapsidated. Dnase activity is neutralized using EDTA or similar, then DNA is liberated from the capsides using proteinase K or similar. DNA is extracted using e.g. phenol-chloroform, and precipitated. Then measuring of the level of DNA may be made using any known method, in particular Southern blot or PCR. IC₅₀ and IC₉₀ may be calculated.

In a second embodiment, the level of virion DNA in the extracellular medium or supernatant is quantified and possibly compared using molecular biology techniques, such as dot blot, Southern blot and quantitative PCR. IC₅₀ and IC₉₀ may be calculated (Inhibition Concentration 50 % or 90 %, say dose of drug which induce a 50 % or 90 % decrease of the measured parameter).

Comparison of the results with standards can be done.

An object of the invention is thus such phenotypic assay method to test the susceptibility of HBV isolates with respect to drugs. In particular, this object is applied to the determination of the susceptibility of the viral strains present in a patient to the available drugs, and then it is possible to adapt antiviral therapy to the virological situation. In a particular embodiment, the method of the invention comprises the following steps:
1) DNA extraction from serum of a patient
2) PCR amplification
3) Cloning into type-I or type-II vectors
4) Transfection of the vectors in cells, e.g. Huh7 cells, and transfer in 96 well plaques
5) Treatment with antiviral to be evaluated
6) Quantitative PCRs and determination of IC₅₀ and IC₉₀
7) Comparison with standards.

Another object of the invention is to use this phenotypic assay method for testing the antiviral activity of current and future drugs to HBV strains or to HBV field isolates. In a particular embodiment, the method of the invention comprises the following steps:
1) Possibly DNA extraction from serum of a patient or from a viral culture
2) PCR amplification
3) Cloning into type-I or type-II vector
4) Transfection of the vectors in cells, e.g. Huh7 cells, or transduction of cells via baculovirus, or production of a stable cell line, and e.g. transfer in 96 well plaques
5) Treatment with antiviral to be evaluated
6) Quantitative PCRs and determination of IC₅₀ and IC₉₀
7) Comparison with standards.

In the method according to the invention, when all or part of the clones obtained from one sample is used to transfect or transduce cells, a multiclonal analysis is made.

The invention thus allows the screening of drugs. A further object of the invention consists in the pharmaceutical compositions useful against HBV that have been prepared after selection of an active agent with the method of the invention. Another object of the invention is a method for producing a pharmaceutical composition to be used as antiviral drug against HBV, comprising the assay method according to the invention, followed by the admixture of the selected active agent with a pharmaceutically acceptable vehicle or excipient.

Another object of the invention is the method to rapidly clone HBV DNA from patient samples, as disclosed herein.

The present invention is additionally described by the following illustrative, non-limiting examples, which refer to the attached drawings.

### Brief description of the drawings:

Figure 1 is a map of the open circular HBV genome with its open reading frames is represented. The differents primers used in this study are indicated
Figure 2 represents a schematic map of pTriEX-1.1 and derived plasmids pTriEX-Mod and pTriEX-Bsd are given as well as the detailed sequence of the basic core promoter (BCP) of the chicken β-actin gene. The modification of the BCP by insertion of a Not I site is shown. The whole map of circular pTriEX-1.1 is represented on Figure 8.
Figure 3 presents the sequence of the forward primer A used for PCR amplification and the primer binding site at the triple-stranded region of the HBV genome.

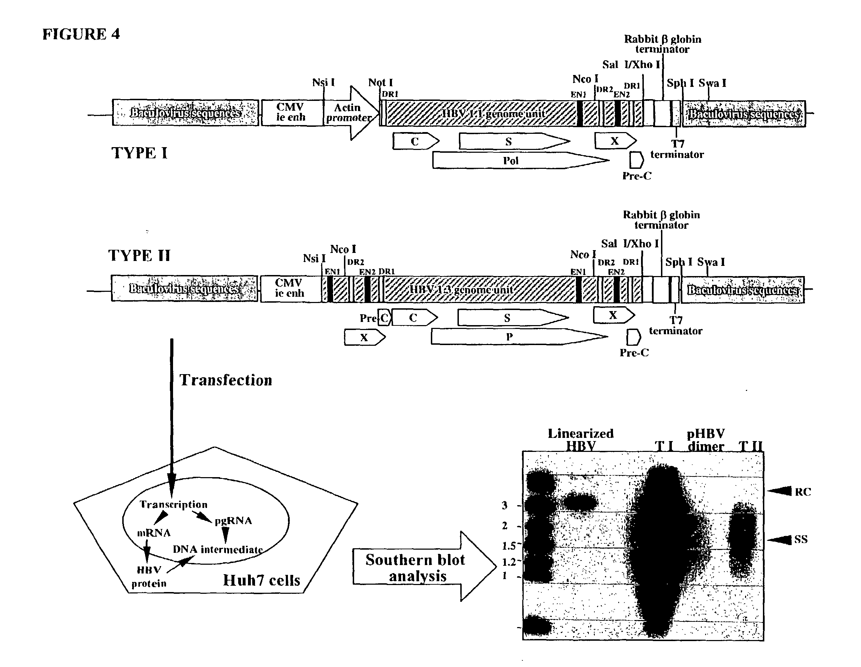

Figure 4 presents type-I and type-II vectors, transfection of Huh7 cells with either type-I, type-II, or pHBVdimer plasmids, and the result of a southern blot analysis of intracellular replicative HBV DNA intermediates. Lanes TI, TII, and pHBVdimer correspond to DNA extracted from viral core particles derived from Huh7 cells that were transfected respectively with type-I, type II, and pHBVdimer circular plasmids. Lane denoted linearized HBV correspond to a 3.2 kb linear double-stranded form of HBV DNA. The position of the relaxed circular (RC) and the single stranded (SS) forms of the HBV DNA genome are indicated on the right of the autoradiography. DNA size markers are indicated on the left.

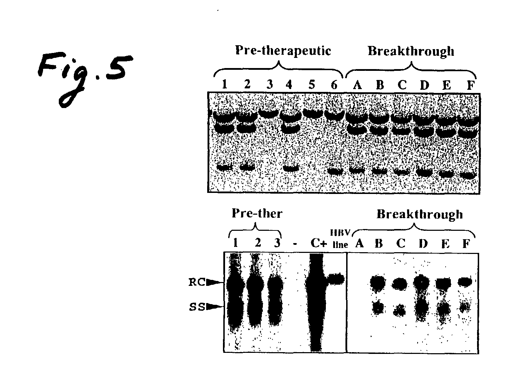

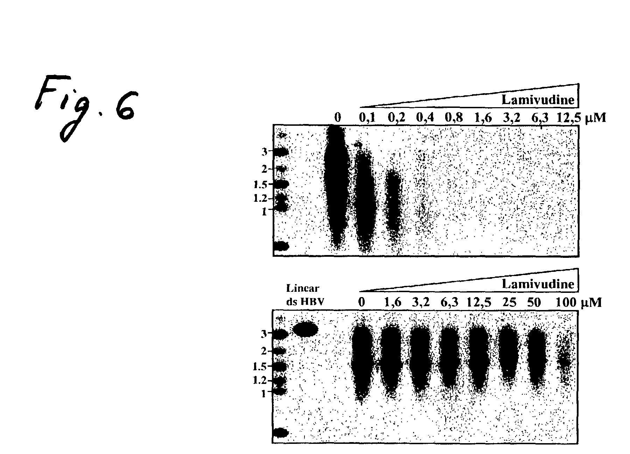

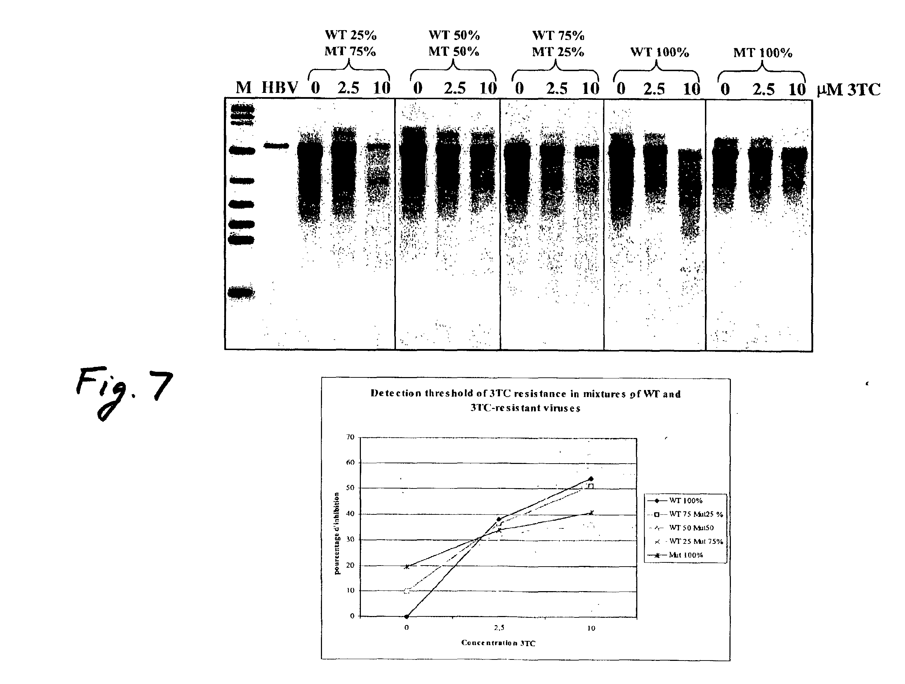

Figure 5 presents cloning performance and replicative status of clones obtained. (A) HBV DNA extracted from serum of patient before therapy (pre-therapeutic) or when viral resistance appear (breakthrough), was PCR amplified using two pairs of primers, i.e. A-B and CD, and cloned in type-I vectors. Positive clones present the restriction pattern found in lanes 1,2, 4, A-F of the ethidium-stained agarose gel presented. Positive clones were used for the transfection of Huh7 cells. A control type-I plasmid carrying a wild type genotypes D HBV genome was used as control (lane C+). Southern blot analysis of intracellular replicative HBV DNA intermediates was performed 4 days post transfection. The position of the relaxed circular (RC) and the single stranded (SS) forms of the HBV DNA genome are indicated on the right of the autoradiography.

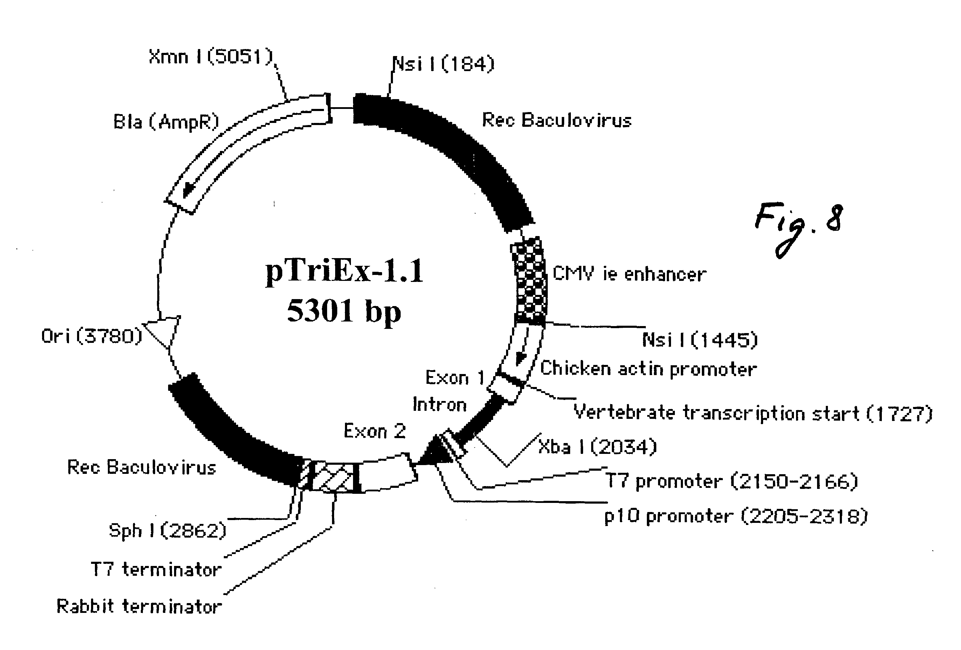

Figure 6 presents two phenotypic drug susceptibility profile after southern blot analysis. Two mixed population of type-I vectors obtained from the cloning of lamivudine-sensitive (top panel) and lamivudine-resistant (botttom panel) HBV genomes have been used for the transfection of Huh7 cells. Cells were treated during 5 days, starting at day 3 post-tranfection, with increasing concentration of lamivudine (shown on the top of each autoradiography). A Southern blot analysis of intracellular replicative HBV DNA intermediates was performed. The autoradiographies corresponding to a sensitive profile (top panel) and a resistant profile (bottom panel) are shown.
Figure 7 presents sensitivity profiles of the phenotypic assay. Mixtures of type-I clones containing either wild type derived HBV genomes or 3TC-resistant derived HBV genomes (containing mutation L528M and M552V) were used for the transfection of Huh7 cells. Cells were left untreated or treated during 5 days, starting at day 3 post-tranfection, with two concentration of lamivudine, i.e. 2.5 or 10 µM. A Southern blot analysis of intracellular replicative HBV DNA intermediates was performed. The composition of the mixture used is indicated on the top of the autoradiography. A phosphoimager analysis of the blot was performed and the results are presented on the graph underneath where the percentage of the inhibition of the replication is represented in function of the amount of lamivudine used.
Figure 8 presents TriEX-1.1 plasmid.

### Examples:

### Materials and Methods

**Plasma samples.** Plasma samples, obtained from chronically HBV-infected individuals, were stored at -20°C until analysis. A reference serum, ACCURUN® 325 HBV DNA positive control manufactured by BBI Diagnotics (Boston, MA), was used for the setting up of some experiments
**Antiviral drugs.** β-L-(-)-2',3'-dideoxy-3'-thiacytidine (3TC, lamivudine), from Glaxo SmithKline.

### Sample preparation and PCR amplifications.

Viral DNA was extracted from 200 µl of serum using three different commercialized kits (Master pure™ Complete DNA and RNA purification kit [Epicentre, Madison, WI], High pure PCR template preparation kit [Roche Diagnostics, Mannheim, Germany], and QIAamp™ UltraSens virus kit [Qiagen, Hilden, Germany]) according to manufacturer's instructions. The final volume of elution or resuspension was 60 µl and 5 to 10 µl were used for subsequent polymerase-chain-reaction (PCR) amplifications. All PCR were performed using a "hot start" procedure with the Expand high fidelity PCR system (Roche Diagnostics, Mannheim, Germany) according to manufacturer's instruction. Four sets of primers were used.

Full length HBV was amplified according to the method described by Günther *et al* (J*.* Virol. 1995, 69, 9: 5437-5444 and J Clin. Microbiol. 1998, 36, 2: 531-538), with the forward primer P1 SEQ ID NO: 22 (5'-CCGGAAAGCTTAT GCTCTTCTTTTTCACCTCTGCCTAATCATC-3') containing *Hind*III/*Sap*| sites, and the reverse primer P2 SEQ ID NO: 23 (5'-CCGGAGAGCTCAT GCTCTTCAAAAAGTTGCATGGTGCTGGTG-3') containing S*a*cl/Sa*p*l sites.

A long PCR product of approximately 2800 bp was amplified by 40 cycles (hot-start; 94°C for 40 s, 55°C for 1 min, 68°C for 3 min with an increment of 2 min after each 10 cycle) with a forward primer A (5'-TGCGCACCGCGGCCGC GCAACTTTTTCACCTCTGCC-3'; SEQ ID NO:1) containing a *Not*l site (see Figure 3) and a reverse primer B (B1 for amplification of genotypes A, B, D, E, and G: 5'-GGCAGCACASCCTAGCAGCCATGG-3', SEQ ID NO:13, or B2 for amplification of genotypes C and F: 5'-GGCAGCACASCCGAGCAGCCATGG-3'; SEQ ID NO: 14) containing a *Nco*l site.

A short PCR product of approximately 665 bp was amplified by 40 cycles (hot-start; 94°C for 30 s, 50°C for 30 s, 72°C for 45 s with an increment of 5 s after each cycle starting from cycle 11) with a forward primer C (C1 for amplification of genotypes A, B, D, E, and G: 5'-ACMTCSTTTCCATGGCTGCTAGG-3', SEQ ID NO:15, or C2 for amplification of genotypes C and F: 5'-ACMTCSTTT CCATGGCTGCTCGG-3'; SEQ ID NO: 16) containing a *Ncol* site and a reverse primer D (D1 containing a *Sph*l site: 5'-CTAAGGGCATGCGATA CAGAGCWGAGGCGG-3', SEQ ID NO:17, or D2 containing a *Sal*l site: 5'-CTAAGGGTCGACGATACAGAGCWGAGGCGG-3'; SEQ ID NO: 18).

An other PCR product of approximately 900 bp was amplified by 40 cycles (hot-start; 94°C for 30 s, 50°C for 30 s, 72°C for 45 s with an increment of 5 s after each cycle starting from cycle 11) with a forward primer E (5'-TAAACAATGCATGAACCTTTACCCCGTTGC-3'; SEQ ID NO: 19) containing a *Nsi*l site and the reverse primer D. The location of these primers in respect with the HBV genome is indicated on Figure 1.

### Cloning of more-than-full-length HBV genome into two different types of vectors.

The pTriEX-1.1 vector (Novagen Inc, Madison, WI., Figure 2), containing multiple promoters and flanking baculovirus sequences, was adapted to the cloning strategy by two modifications to derive pTriEX-Mod. First the Nsil site at position 184 was removed, rendering unique the other *Nsi*l site at position 1445, which is located between the CMV enhancer and the actin basic promoter. Second, a unique *Not*l site was added at position 1717, which is just upstream to the vertebrate transcription start (position 1727) of the vector (Figure 2). The plasmid pTriEX-Mod was further modified by cloning at *Mlu*l site (position 399 in pTriEX-Mod), of the blasticidin cassette that was PCR amplified from pUB-Bsd (Invitrogen Ltd, Paisley, U.K.) comprising the blasticidin gene under the control of human ubiquitin C promoter.

The final vector, pTriEX-Bsd, was used when both transfer into baculoviruses and generation of persistently-HBV-expressing cell line were needed. PCR products generated with primers A-B and E-D (or C-D) were digested by *Not*l/*Nco*l and *Nco*l/*Sal*l respectively and cloned, in one step using standard procedures [Sambrook, J. and D.W. Russell, *Molecular cloning : a laboratory manual.* 3rd ed. 2001, Cold Spring Harbor, N.Y.: Cold Spring Harbor Laboratory Press. 3 v.], between *Not*l/*Xho*l sites of pTriEX-Mod to derive type-I plasmids (Figure 4).

Type-I vector comprising about 1.1 genome unit enables, post-transfection, the synthesis of pre-genomic HBV RNA (pgRNA) under the control of a chicken actin promoter.

Alternatively, PCR product generated with primers E-D was digested by *Nsi*l/*Sal*l and inserted between *Nsi*l/*Xho*l sites of the vector. This intermediate plasmid was subsequently used for the cloning of one HBV genome unit into the unique *Nco*l site located at the level of the ATG of the gene X of HBV. The insert (one HBV genome unit) was obtained by the successive treatments of a P1-P2 full-length PCR product with *Sap*l followed by T4 ligase (circularization) then *Nco*l digestion. This led to type-II vector comprising about 1.3 genome unit, which enables post transfection the synthesis of pgRNA under the control of natural HBV promoter (Figure 4).

### Transfection of HBV DNA.

All cloned DNA were purified by the Qiagen technology, based on silica columns (Qiagen Inc., Valencia, California, USA). Transfection of plasmids into Huh7 cells was performed with Fugene-6 reagent (Roche Diagnostics, Mannheim, Germany) according to manufacturer's instruction. Briefly, Huh7 cells were seeded at a density of 7 x 10⁴ cells/cm² and transfected 12 hours later with 150 ng of plasmid per cm². The medium was changed one hour before transfection, then every day in case of treatment with antiviral.

### Purification of HBV DNA from intracellular core particles and Southern blot analysis.

The cells were washed once with PBS then lysed in 1 ml of lysis buffer (50mM Tris-HCl [pH 8], 1 mM EDTA, 1 % Nonidet P-40) per 35 mm diameter dish. The lysate was incubated on ice for 15 min, and nuclei were pelleted by centrifugation for 1 min at 14,000 rpm. The supernatant was adjusted to 10mM MgCl₂ and treated with 100 µg of DNAse for 1 hour at 37°C. The DNAse activity was stopped by addition of EDTA to a final concentration of 25mM, then proteins were digested with 0.5 mg/ml of proteinase K for at least 2 hours in presence of 1% SDS and 100 mM NaCl. Nucleic acids were phenol-chloroform extracted and iso-propanol precipitated after addition of 10 µg of tRNA. DNAs were separated on 1.2% agarose gel, blotted onto positively-charged Nylon membranes, and hybridized with a ³²P-labelled full-length HBV probe as described previously [Seigneres, B., et al., Hepatology, 2002. **36**(3): p. 710-22.].

### Purification of HBV DNA from extracellular viral particles and quantitative PCR analysis.

Viral DNA was extracted from cell culture medium using either the commercialized QIAamp™ UltraSens virus kit (Qiagen, Hilden, Germany) or by the following procedure. One ml of clarified culture medium was supplemented up to 10%final with polyethylene glycol (PEG), then centrifugated 1 hour at 14,000 rpm in a bench centrifuge at 4°C. The pellet was resuspended in 1 ml of lysis buffer and proceeded as described above for the isolation of HBV DNA from cytoplasmic core particles.

### Results

### Extraction of viral DNA from sera and PCR performance.

Several kits are available for HBV DNA isolation from serum. Three of them have been tested to optimize the quantity/quality of the isolated HBV DNA that will be subsequently used as PCR-template. Some of them imply column-based purification while the other is based on differential precipitation of proteins and nucleic acids. Two hundred microliter of a reference serum were extracted using these different approaches and real time PCR was performed to quantify the amount of DNA obtained. The "Master pure™ Complete DNA and RNA purification kit" yielded higher DNA recovery, with a vDNA (viral DNA) concentration of 2.73 10⁴ copies/µl, compared to the two other column-based techniques, which yielded a concentration of 1.08 10⁴ copies/µl (High pure PCR template preparation kit) and 9.77 10³ copies/µl (QlAamp™ UltraSens virus kit) respectively. To determine whether the quality of the DNA obtained by these techniques was comparable, dilutions (1/10^{th}) of these DNA were used for setting up PCR reactions. Primers P1 and P2, derived from the Gunther's method, were first used for finding out the threshold of the PCR. The PCR was performed using the Expand high fidelity PCR system and the PCR conditions described previously [Günther et al. 1998 and 1995 cited above].

The vDNA obtained with QIAamp™ UltraSens virus kit was the best in terms of quality of the DNA, as a PCR product was visible in an ethidium bromide-stained gel when as low as 5 copies of genome were used as initial template. According to the extraction procedure used (see Materials and Methods), these 5 copies corresponded to a theoretical serum titer of 300 genomes/ml. To obtain the same amount of amplicon using DNA extracted with the Master pure™ Complete DNA and RNA purification kit, 20 copies of genome/reaction (corresponding to a theoretical serum titer of 1200 genomes/ml) were necessary, indicating a lower quality of this DNA.

### Construction of type I vectors:

As described above, a pTriEx-1.1 (Novagen) plasmid was engineered by introduction of a *Not*l site at the level of the +1 of transcription of the chicken actin promoter (Figure 2). No modification of the promoter activity was observed for the modified plasmid, i.e. pTriEX-Mod, as measured by reporter gene expression. Primer A located at the +1 of transcription of the pgRNA contains a *Not*l tail (Figure 3). This primer A was used in combination with the reverse primer B located at the level of the ATG of HBV X gene to set up a PCR reaction. A conserved *Nco*l site, essential for the cloning strategy, overlaps this ATG.

The PCR products obtained with the two pairs of primers A-B and C-D2 (or E-D2) were digested with *Not*l*lNco*l and *Nco*l/*Sal*l and simultaneously cloned into pTriEX-Mod to derive types-I vectors (Figure 4). This fusion resulted in the exclusive production in transfected-hepatoma cells of HBV pgRNA at a steady level regardless of the information contained in the 1.1 unit of genome cloned altogether. In average, between 50 and 75% of the clones obtained presented the expected restriction pattern and more than 75% of these clones led to formation of DNA intermediate upon cell transfection as verified by Southern blot analysis (Figure 5). Taken together, these data indicate that when 40 cycles of PCR are used for generating inserts A-B and C-D, between 40% to 65% of the resulting types I clones have both the expected restriction pattern and are replication-competent.

Within hepatoma cells, type-I vector enables the synthesis of pgRNA at a level that is determined by the chicken actin promoter strength. This type of vector is of interest for the analysis of event of the viral cycle occurring post-transcription. It can be use for instance for characterizing mutations in the polymerase gene, which are associated with resistance to nucleoside/nucleotide analogues.

### Construction of type II vectors:

A second cloning strategy was designed to construct type-II vectors in which the synthesis of pgRNA is kept under homologous promoter. Two cloning steps were necessary to derive these vectors containing 1.3 unit of HBV genome.

First the PCR product of 900 bp obtained with primers E-D2 was double digested with *Nsil*/*Sal*l and cloned into pTriEX-Mod opened by *Nsi*l and *Xho*l. The unique *Nco*l site of the derived intermediate plasmid was then used for cloning one HBV genome unit which was generated by the successive treatment of a P1-P2-derived PCR product with *Sap*l, then T4 DNA ligase, and *Nco*l (Figure 4).

The type-II vector allows to study transcriptional properties of HBV mutants, such as core-promoter mutants, or to evaluate in which the intrinsic level of synthesis of pgRNA had an impact on the phenotype observed.

### Comparison of the amounts of the intracellular replicative intermediates observed in hepatoma Huh7 cells after transfection with type I, type II, head-to-tail tandemerized HBV genomes or HBV genomes with Sapl sticky ends.

The genome of a well defined HBV genotypes D (serotype ayw) was cloned i) into type-I and type-II vectors, ii) as head-to-tail tamdem into pUC vector, or iii) according to Gunther's method into pUC vector. These four types of construction were used for transfecting hepatoma Huh7 cells.

The HBV genome cloned by Günther's method was first released by *Sap*l digestion before the transfection. The same amount of DNA (equivalent HBV genome) was used. As expected, due to presence of the strong actin promoter, the type-I vector gave rise to the highest level of intracellular replicative intermediates (Figure 4), thus confirming the usefulness of such vector for post transcriptionnal phenotypic studies. Type-II vector gave rise to higher level of intracellular replicative intermediates than both head-to-tail tandem and Gunther's type constructions, suggesting that type-II vector may be used as alternative of type-I vector in a phenotypic assay.

### Description and calibration of drug susceptibility assay.

Huh7 cells were seeded in 35mm ⌀ dishes (7. 10⁵ cells/dish) and allowed to adhere for 6 hours. Cells were transfected with 1.5 µg of a mixture of at least 10 clones of type-I vectors obtained from the cloning of HBV from a serum to be tested.

This multiclonal approach was meant to mirror the viral quasispecies. The day after, the medium was replaced by fresh medium containing different concentrations of the drug to be tested (e.g. lamivudine in the example given). Treatment with drug was renewed everyday for four days. Then cells were lysed as described above and a Southern blot analysis was performed to measure the amount of intracellular replicative intermediates synthesized under drug pressure and determine the IC₅₀. Experiments were performed in triplicate. On the example given (Figure 6), for a wild type HBV genomes population the IC₅₀ of lamivudine was estimated less than 100 nM while it increased to more than 50 µM for a viral population cloned from patient who had a viral breakthrough under lamivudine (3TC) treatment.

To get insight on the sensitivity of the assay, incremental mixtures of plasmids containing either wild type derived HBV genomes or 3TC-resistant derived HBV genomes (containing mutation L528M and M552V [Seigneres et al., 2002 supra]) were used for cell transfection. The results of a Southern blot as well as the scan-densitometry analysis are presented on Figure 7. These data demonstrate that the assay readily distinguished mixtures that comprised 25, 50, or 75% resistant virus from the samples with 100% drug sensitive or 100% drug-resistant HBV genomes.

Such modelisation could allows one to compare the IC₅₀ of a treated patient sample to detect a possible breakthrough.

### Various promoters useful in the invention

Are represented below a partial nucleotide sequence of known promoters. The 3' terminal part thereof is represented with the +1 of transcription in bold and underlined (the nucleotide on the left is noted -1) and the TATA box also in bold when present. For each promoter, a location for creating a restriction site is indicated, as well as the A primer(s) which can be used therewith, in accordance with the first embodiment of the invention.

### Basic promoter sequence of Chicken β actin gene:

SEQ ID NO: 24
CGGCCCTATAAAAAGCGAAGCGCGCGGCGGGCGGGAGTCGCT

| Restriction site | Will replace nucleotides | Primer A SEQ ID NO: |
|---|---|---|
| *Notl* | -10 to -3 | 1 |
| *Asc*l | -8 to -1 | 2 |
| Sse8387 l | -10 to -3 | 3 |
| Pmel | -8 to -1 | 4 |

### Basic promoter sequence of CMV-IE gene:

SEQ ID NO: 25
TATATAAGCAGAGCTCGTTTAGTGAACCGTCAGA

| Restriction site | Will replace nucleotides | Primer A SEQ ID NO: |
|---|---|---|
| *Notl* | -9 to -2 | 5 |
| *Asc*l | -8 to -1 | 2 |
| *Sse*8387 l | -9 to -2 | 6 |
| Pmel | -8 to -1 | 7 |

### Basic promoter sequence of human ubiquitin C (hUb) gene:

SEQ ID NO: 26
TATATAAGGACGCGCCGGGTGTGGCACAGCTAGT

| Restriction site | Will replace nucleotides | Primer A SEQ ID NO: |
|---|---|---|
| *Not*l | -11 to -4 | 1 |
| *Asc*l | -9 to -2 | 8 |
| Sse8387 l | -9 to -2 | 6 |

### Basic promoter sequence of human EF-1α gene:

SEQ ID NO: 27
TATATAAGTGCAGTAGTCGCCGTGAACGTTCTT

| Restriction site | Will replace nucleotides | Primer A SEQ ID NO: |
|---|---|---|
| *Notl* | -9 to -2 | 1 |
| *Asc*l | -10 to -3 | 9 |
| *Sse*8387 \| | -10 to -3 | 10 |

### Basic promoter sequence of early promoter of SV40 (GC rich -TATA less promoter):

SEQ ID NO: 28
GACTAATTTTTTTATTTATGCAGAGGCCGAGGC

| Restriction site | Will replace nucleotides | Primer A SEQ ID NO: |
|---|---|---|
| *Notl* | -9 to -2 | 1 |
| *Asc*l | -10 to -3 | 11 |
| *Sse*8387 \| | -10 to -3 | 3 |

### Basic promoter sequence of RSV (Rous Sarcoma Virus) LTR:

SEQ ID NO: 29
TATTTAAGTGCCTAGCTCGATACAATAAACGCC

| Restriction site | Will replace nucleotides | Primer A SEQ ID NO: |
|---|---|---|
| Pmel | -8 to -1 | 7 |
| *Pac*l | -9 to -2 | 12 |
| *Sse*8387l \| | -9 to -2 | 6 |

Each sequence of basic promoter modified by any one of the restiction sites as indicated in the attached table is an object of the invention, as well as each promoter containing that sequence so modified, and each vector containing that promoter.

It should be understood that the invention defined by the attached claims is not limited to the particular embodiments mentioned in the foregoing description but includes variants which do not emerge from the scope or the spirit of the present invention.

## Claims

1. A method for measuring the replication capacity of HBV, e.g. HBV present in a biological sample, possibly in the presence of a pharmaceutical product, in particular an antiviral agent, the method comprising:
(a) possibly extracting nucleic acids contained in the biological sample;
(b) PCR amplification of HBV nucleic acids using at least two primer pairs to obtain at least two amplified HBV genomic fragments representing more-than-full-length HBV genome;
(c) cloning the fragments obtained under (b) into a vector, so producing a vector containing more-than-full-length HBV DNA;
(d) transfecting or transducing susceptible cells with the vector;
(e) culturing the transfected or transduced cells in conditions allowing synthesis of HBV pgRNA from the cloned HBV DNA;
(f) possibly treating the cultured cells with the pharmaceutical product, in particular antiviral agent; and
(g) determination of the replication capacity of the HBV, possibly incidence of the pharmaceutical product, in particular antiviral agent, on viral gene expression and/or viral replication.

2. The method according to claim 1, wherein the more-than-full-length HBV genome comprises HBV nucleotides (from 5' to 3') :
- an about 1 genome unit starting in 5' from and including the nucleotide representing the +1 of transcription to the first nucleotide in 5' of the ATG of the pre-C gene, plus
- a redundant sub-genomic fragment starting from and including the A of the ATG of the pre-C gene and extending to and including the polyA attachment site,
wherein the more-than-full-length HBV genome does not comprise at its 5' end the ATG of the pre-C gene.

3. The method according to claim 2, wherein the more-than-full-length HBV genome comprises from 5' to 3' nucleotides 1818 to 1813 and 1814 to 1960 of an HBV genomic sequence aligned with the sequence as set forth in GenBank AB048704.

4. The method according to claim 2, wherein the more-than-full-length HBV genome comprises from 5' to 3' nucleotides 1816 to 1813 and 1814 to 2016 of an HBV genomic sequence aligned with the sequence as set forth in GenBank AB048704.

5. The method according to any one of claims 2 to 4, wherein in step (b), use is made of a first primer pair and of a second primer pair, comprising each a forward primer and a reverse primer designed to amplify respectively:
- a first fragment comprising a HBV DNA sequence comprising at its 5' end the nucleotide representing the +1 of transcription
- a second fragment comprising a HBV DNA sequence comprising at its 3' end the polyA attachment site
the 3' end of the first fragment and the 5' end of the second fragment being overlapping and preferably comprising a restriction site in the overlapping part.

6. The method according to claim 5, wherein the first primer pair comprises a forward primer A which is partially complementary to the 5' part of the pre-C gene, including the nucleotide representing the +1 of transcription, but does not contain the ATG of the pre-C gene.

7. The method according to claim 6, wherein this forward primer A comprises a restriction site which is not present in the HBV genome.

8. The method according to claim 7, wherein this forward primer A comprises a restriction site chosen among *Not*l*, Asc*l*, Pac*l, *Pme*l and *SSe8387*l.

9. The method according to claim 8, wherein this forward primer A comprises a sequence as set forth in any one of SEQ ID NO: 1 to 12.

10. The method according to any one of claims 5 to 9, wherein the first primer pair comprises a reverse primer B comprising a sequence as set forth in SEQ ID NO: 13 or 14, or a mixture of two reverse primers B comprising a sequence as set forth in SEQ ID NO: 13 and 14, respectively.

11. The method according to any one of claims 5 to 10, wherein the second primer pair comprises a forward primer complementary to a region of HBV DNA which is in 5' with respect to that complementary with the reverse primer of the first primer pair.

12. The method according to claim 11, wherein the forward primer of the second primer pair and the reverse primer of the first primer pair are complementary to HBV genomic regions which overlap and comprise a unique natural restriction site.

13. The method according to claim 12, wherein the forward primer of the second primer pair and the reverse primer of the first primer pair comprise a Ncol site which is complementary to the unique natural Ncol site.

14. The method according to any one of claims 11 to 13, wherein the forward primer comprises a sequence as set forth in SEQ ID NO: 15 or 16 or 19, or is a mixture of two primers comprising a sequence as set forth in SEQ ID NO: 15 and 16, respectively.

15. The method according to any one of claims 5 to 14, wherein the second primer pair comprises a reverse primer D designed to be complementary to a region of the HBV genome which is in 3' of the polyA attachment site.

16. The method according to claim 15, wherein the reverse primer D comprises a sequence as set forth in SEQ ID NO: 17 or 18, or is a mixture of two primers D comprising a sequence as set forth in SEQ ID NO: 17 and 18, respectively.

17. The method according to claim 1, wherein use is made of primers complementary to HBV genomic regions that are well conserved among HBV.

18. The method according to claim 1, wherein the more-than-full-length HBV genome comprises the HBV nucleotide sequence of enhancer 1, a unique *Nco*l site which is situated at the level of the ATG of the X gene, and a polyA attachment site.

19. The method according to claim 18, wherein in step (b), use is made of a first primer pair and of a second primer pair, comprising each a forward and a reverse primers, wherein the first primer pair comprises a forward primer E designed to be complementary to a region of the HBV genome which is in 5' of enhancer 1.

20. The method according to claim 19, wherein primer E comprises a sequence as set forth in SEQ ID NO: 19.

21. The method according to claim 19 or 20, wherein the first primer pair comprises a reverse primer comprising a reverse primer D designed to be complementary to a region of the HBV genome which is in 3' of the polyA attachment site.

22. The method according to claim 21, wherein primer D comprises a sequence as set forth in SEQ ID NO: 17 or 18, or is a mixture of two primers comprising a sequence as set forth in SEQ ID NO: 17 and 18, respectively.

23. The method according to any one of claims 18 to 22, wherein the second primer pair comprises a sense primer comprising a forward primer and a reverse primer allowing to amplify an about one genome unit containing the unique *Nco*l site, with a forward primer designed to be complementary to a region of HBV DNA which is in 5' with respect to that complementary with the reverse primer of the first primer pair, and a reverse primer designed to be complementary to a region of HBV DNA which is in 3' with respect to that complementary with the forward primer of the first primer pair.

24. The method according to claim 23, wherein in the second primer pair, the forward primer comprises a sequence as set forth in SEQ ID NO: 20 or 22 and the reverse primer comprises a sequence as set forth in SEQ ID NO: 21 or 23.

25. The method according to any one of claims 1 to 24, wherein amplification involves from 20 to 60, in particular from 30 to 50, preferably about 40 cycles.

26. The method according to any one of claims 2 to 17, wherein the amplified HBV fragments are cloned into a vector in a one-step cloning procedure.

27. The method according to any one of claims 2 to 17, wherein the vector comprises an heterologous promoter.

28. The method according to claim 27, wherein the +1 of transcription of the heterologous promoter is fused to the +1 of transcription of the HBV fragment.

29. The method according to claim 27 or 28, wherein the promoter is chosen among the group consisting of CMV-IE promoter, human ubiquitine C gene promoter, the promoter of EF-1α gene, the early and late promoters of the SV40 virus, the LTR promoter of the Rous sarcoma virus, cytoskeleton gene promoters, such as, the desmin promoter or the actin promoter, in particular the chicken β-actin promoter.

30. The method according to any one of claims 1 to 29, wherein in step (d), the cells are eucaryotic cells, preferably of hepatocyte origin, preferably cell lines such as hepatoma cells.

31. The method according to claim 30, wherein the cells are directly transfected with the vector.

32. The method according to claim 30, wherein the vector is transferred into baculovirus and then the cells are transduced with the baculovirus.

33. The method according to claim 30, wherein the vector is transferred into a cell line to produce a stable cell line constitutively expressing HBV.

34. The method according to any one of claims 1 to 33, wherein in step (f), the antiviral agent is lamivudine, adefovir dipivoxil, entecavir, emtricitabine, clevudine, telbivudine, tenofovir or any combinations thereof.

35. The method according to any one of claims 1 to 39, wherein in step (f), a molecule is tested as a potential antiviral agent.

36. Polynucleotide useful as primer for HBV amplification, comprising a sequence as set forth in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, or SEQ ID NO: 21.

37. Primer pair for HBV amplification comprising a forward primer comprising (1) a sequence as set forth in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 or SEQ ID NO: 12, and (2) a reverse primer comprising a sequence as set forth in SEQ ID NO: 13 or SEQ ID NO: 14 or a mixture of a reverse primer comprising a sequence as set forth in SEQ ID NO: 13 and a reverse primer comprising a sequence as set forth in SEQ ID NO: 14.

38. Primer pair for HBV amplification comprising (1) a forward primer comprising a sequence as set forth in SEQ ID NO: 15 or SEQ ID NO: 16 or SEQ ID NO: 19, or a mixture of a forward primer comprising a sequence as set forth in SEQ ID NO: 15 and a forward primer comprising a sequence as set forth in SEQ ID NO: 16, and (2) a reverse primer comprising a sequence as set forth in SEQ ID NO: 17 or SEQ ID NO: 18 or a mixture of a reverse primer comprising a sequence as set forth in SEQ ID NO: 17 and a reverse primer comprising a sequence as set forth in SEQ ID NO: 18.

39. Kit for HBV amplification, comprising a primer pair according to claim 47 and a primer pair according to claim 48.

40. Primer pair for HBV amplification comprising (1) a forward primer comprising a sequence as set forth in SEQ ID NO: 19, and (2) a reverse primer comprising a sequence as set forth in SEQ ID NO: 17 or SEQ ID NO: 18 or a mixture of a reverse primer comprising a sequence as set forth in SEQ ID NO: 17 and a reverse primer comprising a sequence as set forth in SEQ ID NO: 18.

41. Primer pair for HBV amplification comprising (1) a forward primer comprising a sequence as set forth in SEQ ID NO: 20 or 22, and (2) an reverse primer comprising a sequence as set forth in SEQ ID NO: 21 or 23.

42. Kit for HBV amplification, comprising a primer pair according to claim 50 and a primer pair according to claim 51.

43. Vector comprising:
- a more-than-full length HBV genome as defined in any one of claims 2 to 4, and
- a promoter modified in 5' by the presence of a restriction site in 5' of the +1 of transcription, with the +1 of transcription of the more-than-full length HBV genome and of the promoter being fused, the promoter controlling the synthesis of a pgRNA from the more-than-full length HBV genome post-cell-transfection.

44. Baculovirus or cell line comprising the vector of claim 43.
